Europäisches Patentamt

European Patent Office

Office européen des brevets

(11) Publication number: **0 149 514**
**A2**

(12) # EUROPEAN PATENT APPLICATION

(21) Application number: **85300063.6**

(22) Date of filing: **04.01.85**

(51) Int. Cl.⁴: **C 12 P 1/04**
**C 02 F 1/56**
**//(C12P1/04, C12R1:085, 1:19,**
**1:385, 1:39)**

(30) Priority: **12.01.84 GB 8400816**

(43) Date of publication of application:
**24.07.85 Bulletin 85/30**

(84) Designated Contracting States:
**AT BE CH DE FR GB IT LI LU NL SE**

(71) Applicant: **SIMON-HARTLEY LIMITED**
**Etruria Works**
**Stoke-on-Trent Staffordshire ST4 7BH(GB)**

(72) Inventor: **Wase, Donald Arthur John**
**Green Leys Cottage**
**Bishampton Nr. Pershore Worcestershire(GB)**

(72) Inventor: **Knight, Nicholas John Bough**
**82 Havelock Road**
**Brighton East Sussex(GB)**

(74) Representative: **Ajello, Michael John**
**P.O. Box 25**
**Stockport Cheshire SK3 OXW(GB)**

(54) **Flocculating agent.**

(57) A flocculating agent is produced by growing a micro-organism, e.g. a strain of pseudomonas, in continuous or batch culture under controlled conditions, and the micro-organism thus cultured is allowed or caused to undergo controlled lysis naturally or artificially using heat or a chemical lysing agent. The extent of the lysis is controlled to optimise the production rate and quality of the product agent which may then be stored for use in industrial applications such as water treatment and paper production.

EP 0 149 514 A2

Croydon Printing Company Ltd.

## FLOCCULATING AGENT

THIS INVENTION relates to a method of producing an organic flocculating agent and to an organic flocculating agent produced by such a method.

According to the present invention there is provided a method of producing an organic flocculating agent, comprising the steps of growing a micro-organism in a culture medium, and allowing or causing the thus cultured micro-organism to undergo controlled lysis to such an extent as to produce a material containing high molecular weight -compounds.

According to another aspect of the present invention, there is provided an organic flocculating agent comprising a material containing high molecular weight compounds which are products of a micro-organism which has undergone controlled lysis, in accordance with the aforesaid method.

The micro-organism is typically an aerobic micro-organism which is grown aerobically. Examples of such micro-organisms are strains of pseudomonas, for example Pseudomonas fluorescens or P. putida, strains of Bacillus e.g. B. cereus, and E. coli.

Whilst the mechanism for producing the flocculating agent is not completely understood, it is believed that when the cultured micro-organism undergoes lysis high molecular weight components present in the cells are released and/or decomposition products of high molecular weight are produced. It is believed that such high molecular weight compounds are proteins, nucleic acids and/or polysaccharides and that such materials produce the required flocculating action possibly either according to the polymer bridging theory or according to the electrostatic theory of flocculation.

The efficiency of the flocculating agent may be enhanced by the removal of any low molecular weight polymers or other low molecular weight species. This may be achieved by such methods as filtration or dialysis. Carbohydrates may also be involved.

The micro-organism may be grown in either a continuous culture or a batch culture. For the continuous culture a continuous fermenter is normally used, with a low dilution rate, typically less than 0.03 $hr^{-1}$, and preferably less than 0.02 $hr^{-1}$, if autolysis is to be used. Higher dilution rates are, preferred if lysis is to be effected by chemical, heat or other external treatment, the dilution rate depending upon the organism employed. Cultivation may be effected under the usual conditions of temperature and

- 3 -    0149514

pH, typically at about 25°C and at a pH of about 7.25. However, a temperature in the range of 20 to 45°C (for mesophilic micro-organisms) or 40 to 70°C (for thermophilic micro-organism) and a pH in the range of 4 to 8 (depending upon the micro-organism), may be employed.

In the case of batch cultivation, similar temperatures may be employed at a starting pH in the range 5.5 to 8 depending upon the micro-organism. For batch cultivation the micro-organisms are normally cultivated for 10 to 270 hours preferably 24-230 hours, before harvesting, depending upon the micro-organisms.

Both continuous and batch cultures are grown on media containing minerals, salts and trace elements in a ratio at which production of flocculating agent is optimised. The culture medium will normally contain a source of assimilable carbon e.g. glucose and may also contain protein e.g. peptone. The flocculating agent is actually produced by the controlled lysis step (to be described), but the medium constituents affect the quality of the flocculating agent produced. For example, a continuous culture medium may consist of di-sodium hydrogen orthophosphate at a concentration of 6.55 g/l; sodium dihydrogen orthophosphate at a concentration of 0.92 g/l; ethylenediaminetetraacetic acid diodium salt at a concentration of 0.89 g/l; magnesium sulphate at a concentration of 0.12 g/l; potassium sulphate at a

- 4 -

0149514

concentration of 2.08g/l; ammonium chloride at a concentration of 3.8 g/l; glucose at concentration of 0.05% w/v to 2% w/v, preferably 0.25%w/v to 1%w/v; and, optionally, peptone at a concentration up to 2% w/v, preferably 0.1%w/v to 0.5%w/v.

A batch culture medium may consist of potassium dihydrogen orthophosphate at a concentration of 13.6 g/l; ammonium sulphate at a concentration of 2 g/l; magnesium sulphate at a concentration of 0.2 g/l; potassium hydroxide at a concentration of 3.78g/l; glucose and peptone at concentrations in the ranges described above.

In either batch culture or continuous culture the medium will normally be sterilized before use.

Growth conditions for the production of flocculating agents should be within established growth conditions for the micro-organisms in question. The exact conditions at which optimum flocculating agent production occurs can be readily determined by spot production and testing of the agent.

The controlled lysis step is important as it is in this step that the production of the flocculating agent occurs. Controlled lysis may be achieved in three ways; by heat treatment, by the use of a chemical lysing agent, or by

- 5 -        0149514

allowing the cells to lyse naturally (autolysis).

The heat treatment may be effected in the following way. After cultivation, the cells are removed from the culture medium e.g. by centrifuging, typically at 1636 g for about 5 to 25 minutes. After resuspending the cells in deionised water, they are subjected to a heat treatment, typically at a temperature of 50°C to 100°C depending upon the micro-organism, preferably 70°C to 100°C for a period of 5 seconds to 100 minutes, preferably 1 minute to 30 minutes depending upon the temperature, followed by rapidly cooling, typically about 1 - 5 minutes, preferably 2 - 4½ minutes. As a further alternative, heat treatment may be effected whilst the cells are still in the culture medium.

Chemical lysing agents can be used to achieve controlled lysis. An example of a suitable lysing agent is Stericol which is an alcoholic anionic detergent solution containing xylenols. Another example of a suitable lysing agent is a two part reagent system wherein one of the reagents is a TE buffer containing 5% Dow Corning anti-foam R.D. emulsion and 0.1 mg/ml zylene cyanol FF, and the other reagent is 1M NaOH saturated at 20°C with sodium dodecylc sulphate. The cells may be centrifuged as described above in relation to heat treatment, and resuspended in a solution of the lysing agent and then violently agitated for about 1 minute to enhance the action of the lysing agent. The flocculating agent thus produced is preferably treated to remove the lysing agent which would otherwise cause degeneration of the flocculating agent, e.g. by washing with a suitable solvent or by dialysis.

An alternative to either of the above methods is autolysis whereinthe cells are allowed to lyse naturally e.g. by leaving them in the culture medium until they have lysed sufficiently, and then centrifuging the mixture for about 5 minutes at about 3000 rpm to separate the flocculating agent produced. The activity of the flocculating agent may be preserved or fixed by adding a preservative such as sodium benzoate to the agent, typically at a final concentration of not less than 0.1 % w/v. The preservative may be added immediately before, or during, or immediately after the lysing step.

It is envisaged that the flocculating agent of this invention can be used in any industrial application of flocculating agents, such as water treatment and paper production.

Embodiments of the invention using continuous culture growth, will now be described in the following Examples 1 to 3.

EXAMPLE 1

A culture medium was produced experimentally as follows. The following ingredients were individually dissolved, mixed and made up to 4.2 litres using tap water:-

| | |
|---|---|
| $Na_2HPO_4$ ($12H_2O$) | 27.5 g |
| $NaH_2PO_4$ ($2 H_2O$) | 3.875 g |
| EDTA | 3.75 g |
| $MgSO_4$ ($7 H_2O$) | 0.5 g |
| $K_2SO_4$ | 8.75 g |

This solution was then sterilisted at 121°C for 20 minutes under a pressure of 15 psig.

19 g of ammonium chloride were dissolved in 400 ml of tap water and sterilised as above. 27.5 g of dextrose monohydrate were dissolved in 400 ml of tap water and sterilised as described above. The three solutions thus produced were then aseptically combined to produce the culture medium for fermentation. Sterilisation was effected separately in the three solutions because the main constituents would interact to the detriment of the medium if sterilised together. The final C:N ratio was 2.13:1. The tap water provided the necessary trace elements.

The above described culture medium was used to cultivate a culture of _Pseudomonas fluorescens_ (NCIB 9046) in a continuous fermenter. In this example, the fermenter had a working volume of 1 litre, but larger sizes are possible. During cultivation, the sterile medium was pumped continuously into the fermenter and air was introduced through a sterilising filter.

During cultivation, the liquid in the fermenter was stirred and the dissolved oxygen level was maintained at between 80% and 98%. The temperature was maintained at or close to 25°C and the pH was maintained constant at 7.5. The pump was controlled so that the average flow rate of liquid through the fermenter was 17.3 ml per hour, representing a dilution rate of 0.0173 hr$^{-1}$. To ensure that steady state conditions were being maintained, the turbidity of the culture was regularly checked by means of a spectrophotometer using a sample removed from the fermenter. The output from the fermenter was collected in a collection bottle under aseptic conditions so that the entire process was carried out aseptically. Collection was over a period of 241.75 hours, representing an output quantity of 3969.5 mls. In this example, the collection bottle was at ambient temperature.

The above described growing conditions in the fermenter were just sufficient to support the micro-organism once the growing process had reached steady state conditions.

Afterwards, the collection bottle was disconnected from the fermenter and was thereby exposed to the atmosphere. Stericol was added to effect lysis of the microorganism, and the contents were centrifuged for 5 minutes at 3000 rpm to separate the viscous flocculating agent produced. A yield of 175 ml of the viscous flocculating agent was produced and stored in a refrigerator until required for use. The equivalent dry weight solids content of the flocculating agent was found to be 2.653 g, the amount being estimated after drying a sample of the flocculating agent at 160°C for approximately 24 hours. The solid material was found to contain almost 100% protein, with a small amount of carbohydrate. The protein content was measured by the biuret method.

In order to test the flocculating effect of the agent thus produced, a standard sedimentation test was conducted, using a suspension of china clay and deionised water. After shaking the clay/water suspension in a glass tube, the sample to be tested was introduced into the top of the tube in liquid form, the tube then being sealed and gently inverted three times over a period of approximately 15 seconds; after which it was held vertically. The sedimentation time was noted against graduations or marks on the glass. The faster the sedimentation time for a given dry weight, the better the flocculating agent. This sedimentation test is a speedy but relatively simple one and some variance in the results was experienced. It was found, on average, that 0.9 mg (dry solids) of this flocculating agent was required to produce a sedimentation time of 5 seconds. In contrast, in a comparative test conducted using an industrial flocculating agent (Decapol C330P), it was found that 3:37 mg (dry solids) were required to produce a similar sedimentation time.


EXAMPLE 2

Example 1 was repeated using the same equipment but selecting different process parameters as follows:-


Culture medium flow rate was 7.3 ml per hour (dilution rate 0.0073 $hr^{-1}$), duration of effluent collection was 384.3 hours, the dissolved oxygen content was between 84% and 94%, the temperature was between 24°C and 25°C, and the pH was between 7.1 and 7.25. A yield of 215 ml of the liquid flocculating agent was extracted by centrifuging in the manner described in Example 1. The equivalent dry weight of the extracted flocculating agent was found to be 2.564 g. In the biuret test, the solids content of the flocculating agent was found to contain about 36% by weight of protein.


As in the sedimentation test described in Example 1, sedimentation times obtainedwith the flocculating agent of Example 2 were dependent upon the degree of dilution of the as-produced liquid flocculating agent. However, it was found that, on average, 2.17 mg (dry solids) of the flocculating agent of Example 2 were required to produce a sedimentation time of 5 seconds.

EXAMPLE 3

Example 1 was repeated using the same equipment but, in this case, the culture medium flow rate was increased to 25.7 ml per hour giving a dilution rate of 0.025 hr$^{-1}$. A yield of 117 ml of the liquid flocculating agent was extracted by centrifuging.

In the sedimentation test the minimum sedimentation time was greater than 5 seconds and it was found that, on average, 1.75 mg (dry solids of the flocculating agent) was required to produce a sedimentation time of 10 seconds.

The results of examples 1 to 3 are given in the table below.

| EXAMPLE | 1 | 2 | 3 |
|---|---|---|---|
| Culture Medium Flow Rate (ml) | 17.3 | 7.3 | 25.7 |
| Dilution Rate (hr$^{-1}$) | 0.0173 | 0.0073 | 0.025 |
| Agent Yield (ml) | 175 | 215 | 117 |

The lower the dilution rate, the longer the standing time of the effluent. Experimental evidence tends to suggest that doubling of the standing time in the effluent vessel results in more than doubling of the quantity of flocculating agent produced.

It is believed that dilution rates in the range 0.001 to 0.1 $hr^{-1}$ can be employed though a rate below 0.03 $hr^{-1}$ is preferred. The dissolved oxygen content can vary between 2% and 90% depending upon the stage of culture and the culture medium flow rate, but should preferably be not less than 80%. Whilst a temperature of 25°C is preferred, temperatures in the range 20 to 70°C can be employed. Whilst it is preferred to maintain the pH at 7.25, it is possible to employ a pH of 4 to 8 or even higher.

Embodiments of the invention using batch culture growth will now be described in the following Examples 4 to 6.

In these experimental batch culture Examples, the culture medium as specified below was used with minor changes as specified in the individual Examples and with the ingredients being individually dissolved and made up to strength using tap water:-

| | | |
|---|---|---|
| Potassium dihydrogen orthophosphate | | 13.6 g/l |
| Ammonium sulphate | | 2 g/l |
| Magnesium sulphate | | 0.2 g/l |
| Potassium hydroxide | | 3.78 g/l |
| Glucose | varied | 0.05% w/v to 2% w/v |
| Peptone | varied | 0.1% w/v to 2% w/v |

The glucose was sterilised separately from the rest of the constituents. The medium was sterilised at 121°C, 15 psig.

EXAMPLE 4

A 500 ml glass shake flask was prepared with 200 ml of the aforementioned batch culture medium with glucose 0.25% and Peptone 0.25%, and was inoculated with P.fluorescens directly from an agar plate using a sterile loop. The flask was stoppered with a sterilisable polypropylene foam plug which allowed the entry of air and acted as a filter. The culture was grown in an Incubator Shaker at 25°C. A 4 ml sample was removed from the culture after 89 hours of inoculation and subjected to the following treatment to obtain the flocculating agent. A chemical lysing agent, Stericol was used in this example. The sample was centrifuged at 1636 g for 25 mins and the supernatant discarded. The pellet of cells was then resuspended in 0.5 ml of previously produced Stericol solution (0.2 ml of Stericol concentrate + 4.8 ml of deionised water) and violently agitated for 1 minute. Five minutes after the commencement of agitation, the flocculating agent was tested for flocculating ability using the same method as described in Examples 1 to 3.

EXAMPLE 5

A batch culture was set up as in Example 4 containing 0.25% w/v glucose and 0.25% w/v peptone. After 163 hours of cultivation at 25°C, the 200 ml culture was harvested and then centrifuged at 1636 g for 20 minutes. A pellet of cells was formed which was resuspended in 25 ml of deionised water. The suspension was allowed to stand for 30 minutes after which it was briefly but violently agitated to complete the suspension and transferred to a glass boiling tube. This concentrated suspension of cells was then immersed in a water bath at 90°C for 5 minutes after which it was cooled under running water. Heat therefore acted as a lysing agent, and the action was stopped abruptly with the running water.

The material produced was very viscous, so it was diluted (4 ml of sample + 16 ml of deionised water) and sedimentation tested as above immediately after cooling.

EXAMPLE 6

The same culture medium as in Example 5 was used and the culture was grown for 211 hours after which the flask was immersed in a boiling water bath for 3 minutes.

The culture went translucent and gelatinous, and 30 ml of this material was mixed with 30 ml of 10% sodium benzoate solution, producing a solution of flocculating agent in 5% w/v sodium benzoate solution. This solution, and the gelatinous material untreated with sodium benzoate were then sedimentation tested and the different results obtained showed that the sodium benzoate acted as a fixing and preservative agent, preventing further lysis. By controlling the lysis in this manner a superior agent as produced.

Experiments also showed that other micro-organisms such as E.coli (NClB10000) and B.cereus (BRL 2401), can be used by selection of the process conditions suitable for the particular species, and these other species can also be processed using a chemical lysing agent, or heat as required and appropriate for each case.

Instead of storing the product flocculating agent in a refrigerator or treating it with a preservative prior to use as in the above examples, it is envisaged that alternative storage methods could be used, including freezing, spray-drying, freeze-drying, vacuum-drying, storage in formalin, and storage in alcohol. For example, it has been found that the flocculating agent can be successfully stored for at least 17 months with virtually no deterioration by freezing at about -10°C. The use of a lysing agent can reduce the production time for the flocculating agent by speeding up the decomposition of the micro-organism. However, since the efficiency of the flocculating agent is dependent upon the production of high molecular weight compounds (probably proteins) it is important to prevent decomposition of such high molecular weight compounds and so it is necessary to control the effect of the lysing agent so as to limit the decomposition. In the case where the culture is disinfected with a lysing agent e.g. a phenolic disinfectant such as stericol, it is possible to limit decomposition by removal or by chemical neutralisation of the lysing agent e.g. by washing the lysing agent out with water, alcohol or acetone.

## CLAIMS

1.      A method of producing an organic flocculating agent, comprising the steps of growing a micro-organism in a culture medium, and allowing or causing the thus cultured micro-organism to undergo controlled lysis to such an extent as to produce a material containing high molecular weight compounds.

2.      A method according to Claim 1, wherein the micro-organism is grown in a continuous culture in which the temperature, pH and dilution rate are maintained within predetermined ranges.

3.      A method according to Claim 2, wherein the temperature is maintained within the range of 20° to 70°C, the pH is maintained in the range 4 to 8 and the dilution rate is in the range 0.001 to 0.1 hr$^{-1}$.

4.      A method according to Claim 1, wherein during growth of micro-organism, the dissolved oxygen level of the culture medium is maintained between 80% and 98%.

5.      A method according to Claim 1, wherein the micro-organism is grown in a batch culture in which the culture medium is inoculated and then agitated for a pre-determined period.

6.        A method according to Claim 1, wherein said controlled lysis is effected by a chemical lysing agent.

7.        A method according to Claim 6, wherein said lysing agent includes a detergent solution containing Xylenols, the components being agitated for a predetermined period, said lysing agent being subsequently removed by, for example, washing, to discontinue lysis.

8.        A method according to Claim 1, wherein said controlled lysis is effected by subjecting the micro-organism to an elevated temperature, in the range 50° to 100°C, for a pre-determined period, followed by rapid cooling.

9.        A method according to Claim 1, wherein said controlled lysis is effected by leaving the micro-organism to lyse naturally for a predetermined period and subsequently fixing same using a preservative agent.

10.       A method according to Claim 1, wherein said controlled lysis is discontinued and fixed using a preservative agent.

11.       A method according to Claim 9, or Claim 10, wherein said preservative agent is sodium benzoate.

12.    A method according to Claim 1, wherein said culture medium contains assimilable carbon and said microorganism is removed from said culture medium by centrifuging prior to said controlled lysis step.

13.    A method according to Claim 1, wherein said high molecular weight compounds contain proteins, and low molecular weight compounds are removed from the product of said controlled lysis.

14.    A method according to Claim 1, wherein said microorganism is a strain of pseudomonas.

15.    An organic flocculating agent comprising a material containing high molecular weight compounds and produced by the method according to any one of Claims 1 to 14.